# EUROPÄISCHE PATENTSCHRIFT

(11) **EP 3 204 053 B1**
(45) Veröffentlichungstag und Bekanntmachung des Hinweises auf die Patenterteilung: **06.07.2022**
(21) Anmeldenummer: 15781052.4
(22) Anmeldetag: 06.10.2015
(51) Int. Cl.: A61L 2/16, A01N 25/34, C11D 17/04

(54) **VERFAHREN UND BEHANDLUNGSVORRICHTUNG ZUR DAUERHAFTEN BESEITIGUNG VON SCHIMMELPILZ**
METHOD AND TREATMENT DEVICE FOR PERMANENT REMOVAL OF MILDEW
PROCÉDÉ ET DISPOSITIF DE TRAITEMENT POUR L'ÉLIMINATION PERMANENTE DES MOISISSURES

(30) Priorität: 06.10.2014 DE 102014114444
(43) Veröffentlichungstag der Anmeldung: 16.08.2017
(73) Patentinhaber: Böhmer, Haico, 64331 Weiterstadt (DE)
(72) Erfinder: Böhmer, Haico, 64331 Weiterstadt (DE)
(74) Vertreter: Habermann Intellectual Property Partnerschaft von Patentanwälten mbB
(86) Internationale Anmeldenummer: PCT/EP2015/073007
(87) Internationale Veröffentlichungsnummer: WO 2016/055449

(56) Entgegenhaltungen:
- EP-A1- 1 125 496
- WO-A1-2007/135424
- DE-A1- 19 955 795
- DE-U1-202012 003 521

## Beschreibung

Die Erfindung betrifft ein Verfahren zur dauerhaften Beseitigung von Schimmelpilz an einer Fläche, wobei in einem Anordnungsschritt eine Behandlungsvorrichtung aus einem gelartigen Antischimmelpilzmittel und einem sporendichten Abdeckteil an einer zu behandelnden Fläche angeordnet wird.

Bei herkömmlichen und bereits aus der Praxis bekannten Verfahren zur dauerhaften Beseitigung von Schimmelpilz wird auf eine mit einem Schimmelpilz befallene Fläche, wie beispielsweise eine Wand oder eine Fliesenfläche, ein Antischimmelmittel aufgetragen. Das Antischimmelmittel beinhaltet dabei gegen den Schimmelpilz wirkende Stoffe, die über eine längere Zeit auf den Schimmelpilz einwirken müssen. Nach einer von dem jeweils verwendeten Antischimmelmittel abhängigen Einwirkzeit, die meistens einige Stunden beträgt, werden das Antischimmelmittel und der Schimmelpilz mechanisch, beispielsweise durch Abtragen, von der Fläche entfernt.

Durch eine mechanische Einwirkung des Antischimmelmittels auf die Fläche beim Auftragen sowie durch das mechanische Abtragen des Schimmelpilzes verbreiten sich dabei die Sporen des Schimmelpilzes meistens großräumig in der Umgebung. Bei einer Schimmelpilzbeseitigung in geschlossenen Räumen verteilen sich die Sporen in der Raumluft und können das Wohlbefinden und die Gesundheit von in den Räumen befindlichen Personen beeinträchtigen, bzw. allergische Reaktionen auslösen. Zudem erhöht sich das Risiko erheblich, dass sich die Sporen an einer anderen Stelle wieder festsetzen und einen erneuten Befall mit dem Schimmelpilz hervorrufen. Aus EP 1 125 496 A1 ist ein Sanierungsmittel zur Behandlung von Schimmelpilzen bekannt, welches auf oder in die schimmelpilzbefallenen Stellen appliziert wird und einen Schutzfilm bilden soll.

Eine in WO 2007/135424 A1 beschriebene Anordnung mehrerer Schichten kann auf eine Oberfläche aufgebracht bzw. aufgeklebt werden. In der Schichtenanordnung ist ein antibakterielles und gegebenenfalls auch pilztötendes Fluid enthalten, welches bei einem Druckkontakt aus der Schichtenanordnung herausgedrückt wird und abgegeben werden kann. Diese Schichtenanordnung kann beispielsweise auf Türklinken oder Griffe aufgebracht werden, um das Übertragen von Krankheitserregern oder Kontaktkontaminationen zu verhindern.

Zusätzlich kann während der Einwirkung auf die mit dem Schimmelpilz befallene Fläche das Antischimmelmittel verdunsten, so dass neben den gesundheitsschädlichen Stoffen, die eingeatmet werden können, auch eine Geruchsbelastung stattfindet. Des Weiteren kann bei einer zu kleinen aufgetragenen Menge des Antischimmelmittels die Verdunstung des Antischimmelmittels dazu führen, dass der Schimmelpilz nicht komplett beseitigt werden kann und eine erneute mehrstündige Behandlung der mit dem Schimmelpilz befallenen Fläche notwendig wird. In DE 20 2012 003 521 U1 ist ein Wirkstoffband beschrieben, welches einen Verdunstungsschutz aufweist, der auf einem einen Wirkstoff enthaltenden Trägermaterial angeordnet ist.

Es wird als eine Aufgabe der vorliegenden Erfindung angesehen ein Verfahren zur dauerhaften Beseitigung von Schimmelpilz derart auszugestalten, dass eine Verbreitung von Schimmelpilzsporen während des Verfahrens sowie eine Geruchsbelastung verhindert werden und dass eine sichere Beseitigung von Schimmelpilz auf einer Fläche gewährleistet wird.

Diese Aufgabe wird erfindungsgemäß dadurch gelöst, in dem Anordnungsschritt das Antischimmelpilzmittel in einem Raster auf die zu behandelnde Fläche aufgetragen wird und anschließend mit dem Abdeckteil abgedeckt wird, und dass nach dem Anordnungsschritt in einem Nachbehandlungsschritt durch ein Druckstück ein Druck auf das Abdeckteil ausgeübt wird, so dass das Antischimmelpilzmittel unter dem Abdeckteil verteilt wird und an der zu behandelnden Fläche anhaften kann.

Durch das gelartige Antischimmelpilzmittel wird ermöglicht, dass keine Verdunstung des Antischimmelpilzmittels während des Verfahrens stattfindet und somit eine dadurch verursachte Geruchsbelastung reduziert wird. Des Weiteren wird ein unerwünschtes Zerfließen oder Verlaufen des Antischimmelpilzmittels auf der zu behandelnden Fläche und insbesondere auf einer Deckfläche verhindert, wodurch das erfindungsgemäße Verfahren auf einfache Weise durchgeführt werden kann. Des Weiteren wird durch eine reduzierte Verdunstung des Antischimmelpilzmittels ermöglicht, dass die Behandlungszeit der Fläche mit dem Antischimmelpilzmittel genau eingehalten werden kann und dadurch eine sichere Beseitigung von Schimmelpilz auf der zu behandelnden Fläche gewährleistet wird.

Das auf dem Antischimmelpilzmittel angeordnete Abdeckteil ist aus einem sporendichten Material hergestellt, beispielsweise aus einer Kunststofffolie, so dass sich während des Verfahrens keine Schimmelpilzsporen in der Luft verbreiten können. Auch das gelartige Antischimmelpilzmittel reduziert bereits bei dem Auftragen eine Verbreitung der Schimmelpilzsporen in der Luft. Das Abdeckteil ermöglich auch die Verdunstung des gelartigen Antischimmelpilzmittels zu reduzieren, wodurch ein Austrocknen des Antischimmelpilzmittels sowie eine Geruchbelastung verhindert werden und eine sichere Beseitigung des Schimmelpilzes auf der Fläche vorgenommen werden kann.

In dem erfindungsgemäßen Verfahren wird die Behandlungsvorrichtung in dem Anordnungsschritt auf der zu behandelnden Fläche angeordnet und erfindungsgemäß kann anschließend in dem Nachbehandlungsschritt das Antischimmelpilzmittel auf der Fläche verteilt werden. Dazu wird mit einem Druckstück, das beispielsweise eine Rollwalze, ein Rakel, ein Schwamm oder ein Andruckklotz sein kann, Druck auf das Abdeckteil und somit auf das darunter befindliche Antischimmelpilzmittel ausgeübt, so dass das gelartige Antischimmelpilzmittel unter dem Abdeckteil auf der zu behandelnden Fläche zerfließt bzw. angedrückt wird und sich gleichmäßig auf der Fläche verteilen kann. Es ist für einen Benutzer der Behandlungsvorrichtung ebenfalls möglich, das Antischimmelpilzmittel mit den Händen, bzw. mit dem Handballen oder dem Handrücken zu verteilen, die dann als Druckstück verwendet werden. Eine auf diese Weise unter dem sporendichten Abdeckteil erzwungene Verteilung des Antischimmelpilzmittels verhindert, dass sich währenddessen und durch die mechanische Einwirkung auf die zu behandelnde Fläche die Schimmelpilzsporen in der Luft verteilen können.

Dabei ist vorgesehen, dass in dem Anordnungsschritt das Antischimmelpilzmittel in einem Raster auf die zu behandelnde Fläche aufgetragen wird und anschließen mit dem Abdeckteil abgedeckt wird. Das Raster kann beispielsweise mäanderförmig, linienförmig oder kreuzförmig ausgebildet sein, so dass eine Auftragung des Antischimmelpilzmittels auf einfache Weise vorgenommen werden kann. Des Weiteren wird dadurch eine genaue Dosierung des Antischimmelpilzmittels möglich, indem beispielsweise je nach dem Abstand der einzelnen Mäanderabschnitte oder Streifen voneinander die Menge des zu verwendeten Antischimmelpilzmittels angepasst werden kann. Nach dem Anordnungsschritt in einem Nachbehandlungsschritt kann dann das Antischimmelpilzmittel unter dem Abdeckteil durch das Druckstück verteilt werden, wobei die Schimmelpilzsporen sich nicht in der Luft verbreiten können.

Durch die Einwirkung auf das Antischimmelpilzmittel während des Nachbehandlungsschritts wird zusätzlich die Haftung des Antischimmelpilzmittels auf der Fläche verbessert. Weiterhin wird die Bildung von Luftblasen verhindert oder durch die Auftragung entstandene Luftblasen in dem Antischimmelpilzmittel wieder ausgedrückt und eine homogene und vollflächige Verteilung des Antischimmelpilzmittels auf der zu behandelnden Fläche erreicht. Dies begünstigt zusätzlich eine sichere Beseitigung des Schimmelpilzes auf der Fläche, ohne dass eine weitere Behandlung oder eine erneute Auftragung des Antischimmelpilzmittels während der Anwendung des Verfahrens notwendig wird.

Erfindungsgemäß ist vorgesehen, dass nach dem Anordnungsschritt in einem Abkapselungsschritt ein Abdeckteilrandbereich des Abdeckteils sporendicht an der Fläche festgelegt wird, so dass sich keine Schimmelpilzsporen in der Luft verbreiten können. Es ist erfindungsgemäß auch vorgesehen, dass in dem Abkapselungsschritt der Abdeckteilrandbereich an einem nicht zu behandelnden Flächenrandbereich der zu behandelnden Fläche haftend und sporendicht festgelegt wird.

Das Abdeckteil kann erfindungsgemäß eine größere Fläche als Antischimmelpilzmittel abdecken, so dass der Abdeckrandbereich mit dem Flächenrandbereich der zu behandelnden Fläche in Kontakt gebracht werden kann. Um eine sporendichte Abkapselung zu ermöglichen, kann in dem Abdeckrandbereich ein Klebestreifen angeordnet sein, der eine sporendichte Haftung des Abdeckteils an der Fläche ermöglicht. Erfindungsgemäß kann der Klebestreifen sowohl bei der Herstellung an dem Abdeckteil vorgefertigt werden als auch als ein getrennt hergestelltes Teil, wie beispielsweise ein nachträglich befestigbares Klebeband, ausgestaltet sein. Der Klebestreifen oder das Klebeband können mit ergänzenden Informationen oder auffallenden Warnhinweisen versehen sein.

Erfindungsgemäß ist auch vorgesehen, dass in dem Anordnungsschritt das Antischimmelpilzmittel und das Abdeckteil der Behandlungsvorrichtung gleichzeitig auf der zu behandelnden Fläche angeordnet werden. So kann beispielsweise das Antischimmelpilzmittel bereits bei der Herstellung auf das Abdeckteil aufgetragen werden und in dem Anordnungsschritt das Antischimmelpilzmittel und das Abdeckteil gleichzeitig auf der zu behandelnde Fläche angeordnet werden. In dem nachfolgenden Nachbehandlungsschritt kann die Behandlungsvorrichtung mit dem Druckstück bearbeitet werden, so dass das Antischimmelpilzmittel an der Fläche anhaften und sich ausbreiten kann. Bei einer derartig ausgestalteten Behandlungsvorrichtung kann die Menge des zu verwendenden Antischimmelpilzmittels genau dosiert werden und dadurch sowohl die Kosten als auch die Umweltbelastung durch die chemischen Bestandteile des Antischimmelpilzmittels reduziert werden.

Es ist vorgesehen, dass in einem Ablöseschritt die Behandlungsvorrichtung von der Fläche entfernt wird. Nach dem Ablösen der Behandlungsvorrichtung können die auf der Fläche haftenden Reste des Antischimmelpilzmittels von der Fläche auf einfache Weise entfernt werden, wobei durch die gelartige Beschaffenheit des Antischimmelmittels die Schimmelpilzsporen in dem Antischimmelpilzmittel haften bleiben und sich nicht verbreiten können.

Das erfindungsgemäße Verfahren kann mit einer nachfolgend beschriebenen Behandlungsvorrichtung zur dauerhaften Beseitigung von Schimmelpilz an einer Fläche durchgeführt werden.

Die Behandlungsvorrichtung weist ein sporendichtes Abdeckteil und ein gelartiges Antischimmelpilzmittel auf, sodass mit der Behandlungsvorrichtung das Verfahren wie oben beschrieben durchgeführt werden kann. Das sporendichte Abdeckteil kann beispielsweise aus einer Kunststofffolie hergestellt sein, so dass während des Verfahrens keine Verbreitung der Schimmelpilzsporen in der Luft stattfindet. Das gelartige Antischimmelpilzmittel ermöglicht zudem eine sichere und genaue Anordnung des Antischimmelpilzmittels an der zu behandelnden Fläche.

Weiterhin kann das Antischimmelpilzmittel ein Treibmittel zum Aufschäumen des Antischimmelpilzmittels enthalten. Das Treibmittel kann nach der Anbringung der Behandlungsvorrichtung beispielsweise durch Wärme aktiviert werden und ein Aufschäumen des Antischimmelpilzmittels bewirken, so dass das Antischimmelpilzmittel in alle Unebenheiten und angrenzenden Hohlräume wie beispielsweise Mauerspalten sowie Risse oder Löcher in einer Wand, einer Decke oder einem Boden eindringt.

Vorteilhafterweise kann vorgesehen sein, dass das Abdeckteil an einem Abdeckteilrandbereich ein sporendichtes Haftmittel aufweist, um das Abdeckteil sporendicht an der zu behandelnden Fläche festlegen zu können. So kann beispielsweise bereits bei der Herstellung des Abdeckteils in dem Abdeckbereich eine Kleberschicht aufgetragen werden oder ein getrennt hergestelltes Haftmittel beispielsweise in Form eines Klebebandes verwendet werden. Die Kleberschicht kann dabei beispielsweise bei vorgefertigten eine bestimmte Größe aufweisenden Behandlungsvorrichtungen verwendet werden, um kleine Flächen mit dem Antischimmelpilzmittel behandelt zu können. Bei den unregelmäßigen oder großen Flächen kann vorteilhaft das Klebeband eingesetzt werden und das Abdeckteil erfindungsgemäß auf die Größe der zu behandelnden Fläche zugeschnitten werden.

Die Behandlungsvorrichtung kann eine Sicherheitsfolie aufweisen, wobei die Sicherheitsfolie an den Abdeckteilrandbereichen des Abdeckteils lösbar festgelegt ist und wobei das Antischimmelpilzmittel zwischen der Sicherheitsfolie und dem Abdeckteil angeordnet ist. Eine derartig ausgestaltete Behandlungsvorrichtung kann beispielsweise an kleinen Flächen verwendet werden, wobei die Sicherheitsfolie ein Austrocknen des Antischimmelpilzmittels verhindert. So kann die Behandlungsvorrichtung beispielsweise bereits vollständig vorgefertigt sein und bei der Anwendung zu gegebener Zeit die Sicherheitsfolie entfernt und das Abdeckteil mit dem Antischimmelpilzmittel an der Fläche angeordnet werden.

Weiterhin kann das Abdeckteil ein Trägerelement aufweisen, in dem das Antischimmelpilzmittel angeordnet werden kann. Das Trägerelement kann beispielsweise ein weicher Stoff sein, das das Antischimmelpilzmittel aufnehmen kann. So wird bei der Behandlungsvorrichtung zusätzlich verhindert, dass das Antischimmelpilzmittel sich unter der Abdeckfolie verlagern kann und die Wirkung des Antischimmelmittels an einzelnen Bereichen der zu behandelnden Fläche nachlässt.

Das Antischimmelpilzmittel kann ein verstoffwechselndes Fungizid mit einer fluoreszierenden oder chemolumineszierenden Substanz enthalten. In den von dem Antischimmelpilzmittel bedeckten Bereichen nimmt der Schimmelpilz während einer Behandlungsdauer das Fungizid mit der fluoreszierenden oder chemolumineszierenden Substanz auf und verstoffwechselt das Fungizid, so dass es allmählich über den gesamten Schimmelpilz verteilt wird. Nach einem ausreichend langen Zeitraum von beispielsweise mehreren Tagen ist die von dem Schimmelpilz aufgenommene fluoreszierende Substanz oder die chemolumineszierende Substanz über weite Bereiche des Schimmelpilzes bzw. über den gesamten Schimmelpilz verteilt. Mit einer geeigneten, beispielsweise optischen Anregung kann die fluoreszierende Substanz und damit die Ausbreitung des Schimmelpilzes sichtbar gemacht werden. Ebenso kann beispielsweise durch Verdunkelung und Aktivierung die chemolumineszierende Substanz sichtbar gemacht und dadurch die Ausbreitung des Schimmelpilzes nachgewiesen werden. Auf diese Weise ist es möglich, gegebenenfalls weitere Bereiche mit einem signifikanten Schimmelpilzbefall zu ermitteln, die zunächst nicht erkennbar waren.

Weitere vorteilhafte Ausgestaltungen des erfindungsgemäßen Verfahrens sowie einer Behandlungsvorrichtung, mit welcher das erfindungsgemäße Verfahren durchgeführt werden kann, werden anhand von in der Zeichnung dargestellten Ausführungsformen näher erläutert. Es zeigt:
- Fig. 1 und 2: eine Seitenansicht und eine Draufsicht einer Behandlungsvorrichtung an einer Fläche in einem Anordnungsschritt;
- Fig. 3 und 4: eine Seitenansicht und eine Draufsicht der in Fig. 1 und Fig. 2 gezeigten Behandlungsvorrichtung nach dem Anordnungsschritt;
- Fig. 5 und 6: eine Seitenansicht und eine Draufsicht der in Fig. 1 und Fig. 2 gezeigten Behandlungsvorrichtung nach einem Abkapselungsschritt;
- Fig. 7 und 8: eine Seitenansicht und eine Draufsicht der in Fig. 1 und Fig. 2 gezeigten Behandlungsvorrichtung in einem Nachbehandlungsschritt;
- Fig. 9 und 10: eine Seitenansicht und eine Draufsicht der in Fig. 1 und Fig. 2 gezeigten Behandlungsvorrichtung nach einem Nachbehandlungsschritt;
- Fig. 11, 12 und 13: verschieden ausgestaltete Behandlungsvorrichtungen.

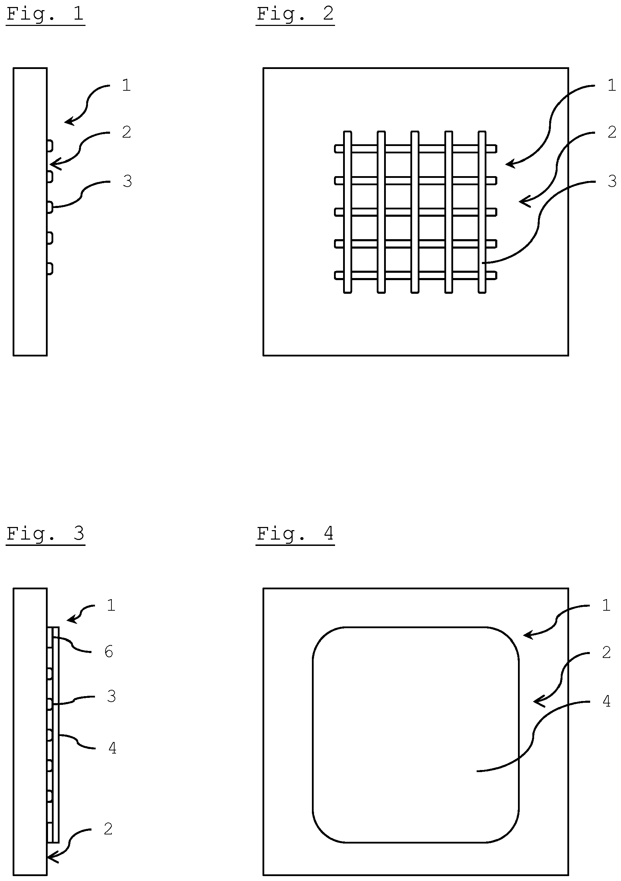

Fig. 1 und Fig. 2 zeigen eine Seitenansicht und eine Draufsicht einer Behandlungsvorrichtung 1 an einer zu behandelnden Fläche 2, wobei ein Antischimmelpilzmittel 3 bereits auf der Fläche 2 aufgetragen ist. Das Antischimmelpilzmittel 3 ist in einem Raster aufgetragen, wobei dadurch eine genaue Dosierung des Antischimmelmittels 3 auf der Fläche 2 möglich ist.

In Fig. 3 und in Fig. 4 ist eine Seitenansicht und eine Draufsicht der Behandlungsvorrichtung 1 nach einem abgeschlossenen Anordnungsschritt gezeigt, wobei ein Abdeckteil 4 auf dem Antischimmelpilzmittel 3 angeordnet ist. Das Abdeckteil 4 weist in einem Abdeckrandbereich 5 ein Haftmittel 6 auf, durch das die Behandlungsvorrichtung 1 an der Fläche 2 festgelegt werden kann.

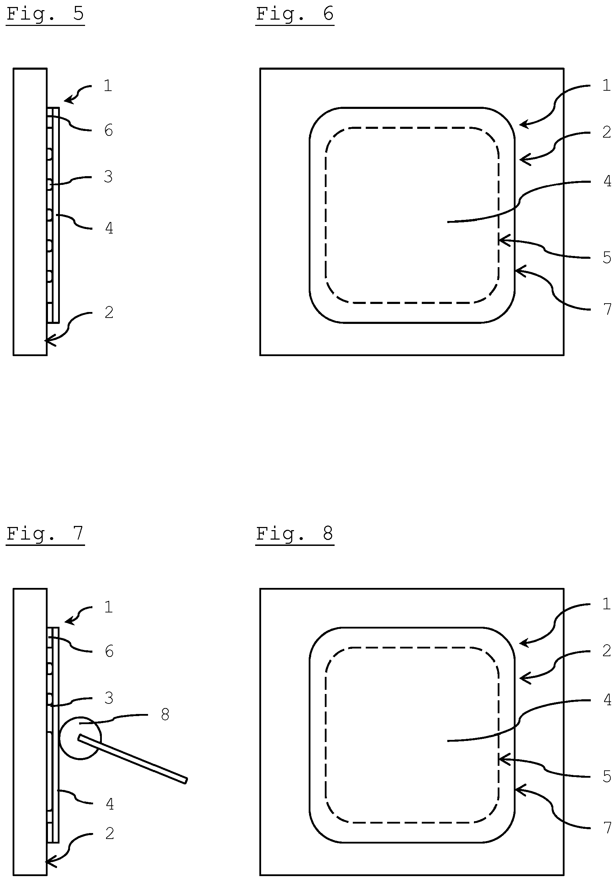

Fig. 5 und Fig. 6 zeigen eine Seitenansicht und eine Draufsicht der in Fig. 1 und Fig. 2 gezeigten Behandlungsvorrichtung 1 nach einem Abkapselungsschritt. Dabei wurde zwischen dem Abdeckteil 4 an dem Abdeckrandbereich 5 und einem Flächenrandbereich 7 der Fläche 2 ein Kontakt hergestellt, so dass die Behandlungsvorrichtung 1 an der Fläche 2 festgelegt ist.

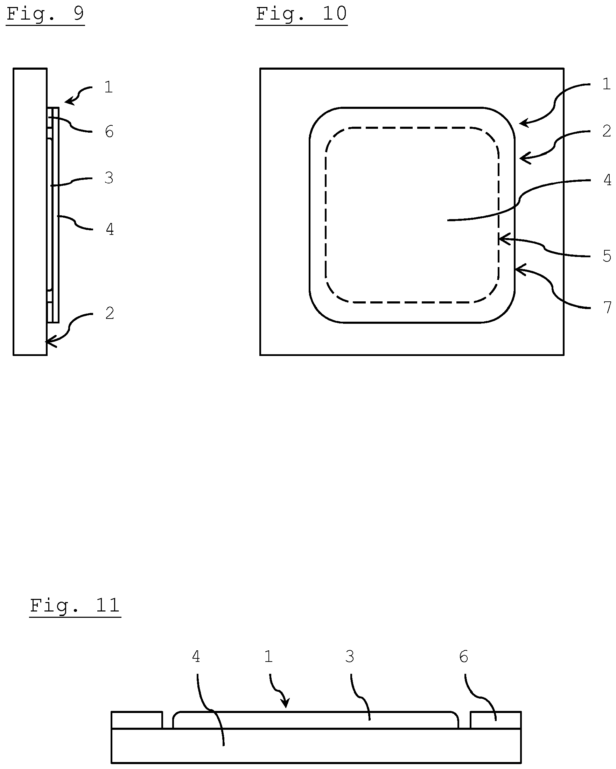

Nach dem Abkapselungsschritt in einem Nachbehandlungsschritt kann nun das Antischimmelpilzmittel 3 unter dem Abdeckteil 4 durch ein Druckstück verteilt werden, wie in Fig. 7 und Fig. 8 gezeigt ist. Als Druckstück kann auch ein Schwamm oder lediglich eine Hand oder ein Arm eines Benutzers zum Verteilen des Antischimmelpilzmittels 3 verwendet werden. Nach dem Nachbehandlungsschritt, wie in Fig. 9 und Fig. 10 gezeigt, ist das Antischimmelpilzmittel 3 gleichmäßig unter dem Abdeckteil 4 verteilt, so dass eine Haftung des Antischimmelpilzmittels 3 an der Fläche 2 verbessert werden und das Antischimmelpilzmittel 3 besser wirken kann.

In Fig. 11 ist eine Seitenansicht der Behandlungsvorrichtung 1 gezeigt, wobei die Behandlungsvorrichtung 1 das Abdeckteil 4, das Antischimmelpilzmittel 3 und das in dem Abdeckrandbereich angeordnete Haftmittel 6 aufweist. Die in Fig. 11 dargestellte Behandlungsvorrichtung 1 kann in einem Schritt an der Fläche 2 angeordnet werden und anschließend in dem Abkapselungsschritt an der Fläche 2 festgelegt werden.

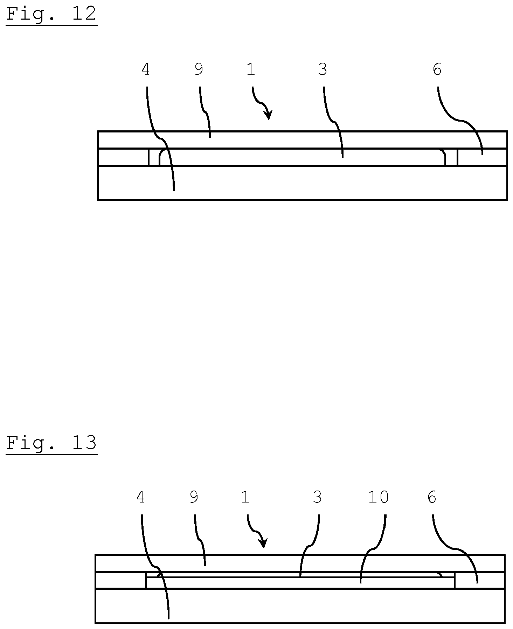

In Fig. 12 ist eine Seitenansicht der Behandlungsvorrichtung 1 dargestellt, die eine Sicherheitsfolie 9 aufweist. Die Sicherheitsfolie 9 gewährleistet, dass das Antischimmelpilzmittel 3 vor der Anwendung nicht von dem Abdeckteil 4 gelöst wird und nicht austrocknen kann. Die in Fig. 12 gezeigte Behandlungsvorrichtung 1 kann am Werk hergestellt sein und bei der Anwendung wird lediglich die Sicherheitsfolie 9 entfernt und die Behandlungsvorrichtung 1 an der Fläche 2 angeordnet und festgelegt.

Eine Seitenansicht der Behandlungsvorrichtung 1, die ein Trägerelement 10 aufweist, ist in Fig. 13 gezeigt. Das Trägerelement 10 ermöglicht eine zusätzliche Festlegung des Antischimmelpilzmittels 3 an dem Abdeckteil 4 und verhindert zusätzlich ein Fließen des Antischimmelpilzmittels 3 an der Fläche 2.

## Patentansprüche

1. Verfahren zur dauerhaften Beseitigung von Schimmelpilz an einer Fläche (2), wobei in einem Anordnungsschritt eine Behandlungsvorrichtung (1) aus einem gelartigen Antischimmelpilzmittel (3) und einem sporendichten Abdeckteil (4) an der zu behandelnden Fläche (2) angeordnet wird, **dadurch gekennzeichnet, dass** in dem Anordnungsschritt das Antischimmelpilzmittel (3) in einem Raster auf die zu behandelnde Fläche (2) aufgetragen wird und anschließend mit dem Abdeckteil (4) abgedeckt wird, und dass nach dem Anordnungsschritt in einem Nachbehandlungsschritt durch ein Druckstück (8) ein Druck auf das Abdeckteil (4) ausgeübt wird, so dass das Antischimmelpilzmittel (3) unter dem Abdeckteil (4) verteilt wird und an der zu behandelnden Fläche (2) anhaften kann.

2. Verfahren nach Anspruch 1, **dadurch gekennzeichnet, dass** nach dem Anordnungsschritt in einem Abkapselungsschritt ein Abdeckteilrandbereich (5) des Abdeckteils (4) sporendicht an der Fläche (2) festgelegt wird, so dass keine Schimmelpilzsporen sich in der Luft verbreiten können.

3. Verfahren nach Anspruch 2, **dadurch gekennzeichnet, dass** in dem Abkapselungsschritt der Abdeckteilrandbereich (5) an einem nicht zu behandelnden Flächenrandbereich (7) der zu behandelnden Fläche (2) haftend und sporendicht angeordnet wird.

4. Verfahren nach einem der vorangehenden Ansprüche, **dadurch gekennzeichnet, dass** in einem Ablöseschritt die Behandlungsvorrichtung (1) von der Fläche (2) entfernt wird.

## Claims

1. Method for the permanent removal of mould on a surface (2), wherein in a set-up step a treatment device (1) made of a gel-like anti-mould agent (3) and a spore-tight cover part (4) is arranged on the surface (2) to be treated, **characterised in that** in the set-up step the anti-mould agent (3) is applied to the surface (2) to be treated in a grid and is then covered with the cover part (4), and that after the set-up step, in a post-treatment step, a pressure is applied to the cover part (4) by means of a pressure piece (8) such that the anti-mould agent (3) is distributed under the cover part (4) and can adhere to the surface (2) to be treated.

2. Method according to claim 1, **characterised in that** after the set-up step, in an encapsulation step, a cover part edge region (5) of the cover part (4) is fixed to the surface (2) in a spore-tight manner so that no mould spores can disseminate in the air.

3. Method according to claim 2, **characterised in that** in the encapsulation step the cover part edge region (5) is arranged in an adherent and spore-tight manner on a surface edge region (7), which is not to be treated, of the surface (2) that is to be treated.

4. Method according to one of the preceding claims, **characterised in that** in a release step the treatment device (1) is removed from the surface (2).

## Revendications

1. Procédé pour éliminer durablement de la moisissure sur une surface (2), dans lequel un dispositif de traitement (1) composé d'un agent anti-moisissures (3) de type gel et d'une partie de recouvrement (4) qui retient les spores est disposé sur la surface (2) à traiter lors d'une étape d'agencement, **caractérisé en ce que** lors de l'étape d'agencement, l'agent anti-moisissures (3) est appliqué en une trame sur la surface (2) à traiter puis est recouvert de la partie de recouvrement (4), et qu'une pression est exercée sur la partie de recouvrement (4) par une pièce de pression (8) après l'étape d'agencement lors d'une étape de post-traitement de sorte que l'agent anti-moisissures (3) est réparti sous la partie de recouvrement (4) et peut adhérer sur la surface (2) à traiter.

2. Procédé selon la revendication 1, **caractérisé en ce qu'**après l'étape d'agencement, une zone de bord de partie de recouvrement (5) de la partie de recouvrement (4) est fixée sur la surface (2) de manière à retenir les spores après l'étape d'agencement lors d'une étape d'encapsulation de sorte qu'aucune spore de moisissure ne peut se propager dans l'air.

3. Procédé selon la revendication 2, **caractérisé en ce que** lors de l'étape d'encapsulation, la zone de bord de partie de recouvrement (5) est disposée de manière à adhérer et de manière à retenir les spores sur une zone de bord de surface (7) qui n'est pas à traiter de la surface (2) à traiter.

4. Procédé selon l'une quelconque des revendications précédentes, **caractérisé en ce que** lors d'une étape de détachement, le dispositif de traitement (1) est retiré de la surface (2).
